# EUROPEAN PATENT SPECIFICATION

(11) **EP 4 444 196 B1**
(45) Date of publication and mention of the grant of the patent: **07.10.2026**
(21) Application number: 23847293.0
(22) Date of filing: 26.07.2023
(51) Int. Cl.: A61B 17/12, A61M 25/00

(54) **SYSTEMS FOR DETACHMENT OF RELEASABLE DEVICES**
SYSTEME ZUR ABLÖSUNG LÖSBARER VORRICHTUNGEN
SYSTÈMES DE DÉTACHEMENT DE DISPOSITIFS LIBÉRABLES

(30) Priority: 26.07.2022 US 202263369499 P
(43) Date of publication of application: 16.10.2024
(73) Proprietor: Life Vascular Devices Biotech, S.L., 08620 Saint Vinenç dels Horts (ES)
(72) Inventor: Lingane, Paul James, Redwood City, CA 94062 (US); Gorospe, Marcelino B., Redwood City, CA 94061 (US)
(74) Representative: ZBM Patents - Zea, Barlocci & Markvardsen
(86) International application number: PCT/US2023/028669
(87) International publication number: WO 2024/025935

(56) References cited:
- US-A- 5 722 989
- US-A1- 2009 163 780
- US-A1- 2012 330 349
- US-A1- 2018 289 925
- US-A1- 2018 289 925
- US-A1- 2019 231 302

## Description

### FIELD

This disclosure is directed to systems for implanting devices in the body of a patient. More particularly, the disclosure relates to systems for thermally severing a connector that couples a releasable device to an end portion of an elongated shaft through the use of a resistor device/heater that is adjacent to or in contact with the connector.

### BACKGROUND

An aneurysm is a localized bulge in the wall of a blood vessel caused by a weakness in the blood vessel wall. As an aneurysm increases in size, the risk of rupture increases. Aneurysms can occur in any artery, with particularly detrimental examples including aneurysms in the brain and abdominal aortic aneurysms. Aneurysms can arise in the heart itself following a heart attack, including both ventricular and atrial septal aneurysms.

Aneurysms may be treated with a variety of methods, the method of treatment particularly depending on the size and location of the aneurysm. Brain aneurysms are often treated using a variety of methods, or a combination of methods, depending on the type of aneurysm and the individual patient. These methods may include microsurgical clipping, endovascular coiling, endovascular stent coiling, artery occlusion and bypass, flow diversion with stents, and tubular retractor systems. Some of these methods involve the use of interventional devices that are delivered endovascularly.

Vascular interventional devices used for these procedures can have a wide variety of configurations, including detachable vasoocclusive balloons and embolus generating vasoocclusive devices. Embolus generating vasoocclusive devices are placed within an aneurysm stemming from the blood vessel to form an embolus within the aneurysm. The devices induce clotting (embolization) of the aneurysm and, in this way, prevent blood from further entering and enlarging the aneurysm. Typically, the device comprises a vasoocclusive coil, such as a helical wire coil having windings which may be dimensioned to engage the walls of the blood vessel. Other less stiff helically coiled devices have been described, as well as those involving woven braids.

The delivery of such vascular interventional devices has been accomplished by a variety of means, including via a catheter in which the device is pushed through an opening at the proximal end of the catheter by a pusher wire used to deploy the device. The device is radio-opaque, and the physician visualizes the position of the device as it is being introduced by using a fluoroscopic X-ray system. In some instances devices are produced in such a way that they will pass through the lumen of a catheter in a linear shape and then take on a complex shape as originally formed after being deployed into the area of interest, such as an aneurysm.

One conventional releasable endovascular therapeutic device used to embolize aneurysms has a polymer thread acting as a tether to attach a coil to a pusher wire. This permits the coil to be pushed and pulled via the pusher wire to position it in the aneurysm. This polymer thread runs adjacent to a miniature electrical heater element at the distal tip of the pusher wire such that when the heater is energized the polymer thread is melted and the coil is released. When the coil is thus detached from the pusher wire, it remains in position in the aneurysm while the pusher wire and heater assembly is withdrawn from the body.

A problem of prior art devices is that the heater element is formed from a coil of metal wire where the resulting resistance has a large tolerance due to variability in the manufacturing process. It is expensive to manufacture and the large tolerance in resistance value means that the amount of power dissipated in the heater also varies by a large amount from device to device. This adds further uncertainty to the resulting temperature of the heater element when energized.

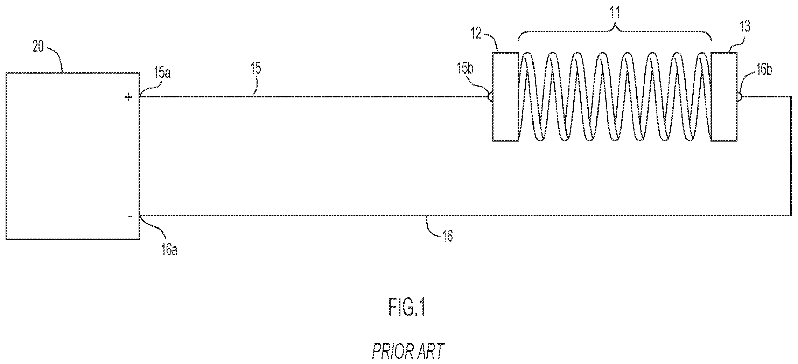

In prior art devices, as shown in FIG. 1, first and second electrically conductive wires 15 and 16 made of copper couple the coil of metal wire 11 to a power source 20. First ends 15a and 16a of wires 15 and 16 are coupled to the power source 20, and second ends 15b and 16b of wires 15 and 16 are respectively coupled to electrical terminals 12 and 13 located at opposite ends of the coil of metal wire 11. In use, at a time when it is desirable to sever the thread located on or adjacent the coil metal wire 11, a controller associated with the power source 20 turns the power source on for a designated period of time to cause a heating of the metal wire 11. The severing of the thread generally involves a melting of the thread.

Patent document US 2009/163780 Al describes a system for quickly detaching an implant and for locating the detachment zone of a detachable implant. Patent document US 5 722 989 A describes a micro-mechanical system for medical procedures in the form of a catheter, a fiberoptic cable being disposed in the catheter. Patent document US 2018/289925 Al describes a catheter comprising a heater control supplying power, a handle, a base catheter with lumen and comprising a proximal section and a distal segment and a variable stiffness element.

### SUMMARY

There is provided a system as defined in claim 1. Other aspects of the system are set forth in the dependent claims.

Devices, systems and methods are provided for delivering a releasable device (e.g. releasable implantable device), where the system comprises an elongated shaft having a proximal end and a distal end, the distal end configured for advancement into the body. A coupling element e.g. a polymeric thread) retains the releasable device near the distal end of the elongated shaft until sufficient thermal energy applied thereto alters the coupling element causing detachment of the releasable device. The coupling element may be any of a number of objects that are severable by the application of heat thereto. A heater (also referred to herein as a "resistor device) disposed within or otherwise coupled to the elongated shaft is configured to apply thermal energy to the coupling element, wherein the thermal energy alters the coupling element in response to the actuation, releasing the implantable device. A controller is configured to deliver an electrical drive signal to alter the state of one or more switches between first and second states in order to respectively couple and decouple the heater from an electrical power source. That is, when the one or more switches are in the first state, electrical power is delivered to the heater and when the one or more switches are in the second state, electrical power is not delivered to the heater.

In some implementations, the heater comprises a coil of metal wire that will heat sufficiently to sever a thread ( e.g. a polymeric thread), or other severable connector, when a sufficient amount of electrical current flows through the coil.

According to other implementations, the heater comprises a surface-mount device resistor (also known as a "chip resistor"). A chip resistor is a passive electronic component that is designed to limit the flow of current. The traditional use of a chip resistor is to lower the voltage or maintain the current constant inside an electronic circuit. Chip resistors are widely used in applications such as automotive and transportation, consumer electronics, industrial and IT and telecommunications. The resistive elements of chip resistors may comprise, for example, thin film resistors, thick film resistors and foil resistors. The present invention uses the chip resistor in a non-traditional way by utilizing its resistive element to convert electrical energy into thermal energy so that the chip resistor functions as a heater.

Thick film and thin film chip resistors used in the electronics industry come in a variety of package/case sizes, such as 0201 (imperial code) / 0510 (metric code) or package/case size of 0402 (imperial code) / 1005 (metric code) or package/case size of 009005 (imperial code) / 03015 (metric code) or smaller. One advantage of using these commercially available resistors as heaters is that they are available with a narrow resistance tolerance, typically +/- 1 % tolerance or better, so that the total heater resistance tolerance can be more tightly controlled.

When detaching an implantable device using a thermal detachment method, it is advantageous to control the heater to a specific temperature *in situ*, rather than relying on a certain amount of energy delivery ( a certain power for a specific time, or a certain voltage or current for a specific time) to raise the temperature of the heater to an approximate temperature to sever the thermally severable connector that tethers the implantable device to its delivery platform. This method is susceptible to variation in the surrounding thermal milieu, causing the temperature of the heater to vary from case to case despite the electrical energy delivered being the same. If a constant voltage or constant current is supplied to the heater the resulting temperature is also susceptible to variation in the electrical resistance of the heater, which is a manufacturing variable that is not perfectly controlled, particularly when the resistive element of the heater is a coiled metal wire.

Prior art designs using coiled metal resistive elements require tuning the heater power and duration: too high a power or too long and the plastic delivery system ( e.g. the surrounding microcatheter/delivery catheter) can be thermally damaged so the introducer has difficulty being pulled out, and too little power or too short duration means it will not always detach. For these reasons, according to one aspect the heater is controlled to a specific temperature or a specific temperature range to ensure the releasable implantable device detaches reliably and does not result in damage to the delivery system.

Ways to heat to a specific temperature include measuring a temperature of the heater in a feedback scheme to control the power going to the heater to drive it to a specific temperature. A feedback scheme is possible to measure the temperature of the heater and then control the power to the heater in order to control its temperature to a setpoint/ target temperature. The target temperature may be a temperature range, such as 200 degrees C ± 20 degrees C.

According to one aspect of the invention, first and second electrically conductive elements (also referred to herein as "electrical conductors") made of dissimilar metals (those having different Seebeck coefficients) are respectively coupled to first and second electrical terminals of the heater. According to some implementations, disposed between the first and second electrically conductive terminals is a resistive element made of an electrically conductive material that heats up when a current passes through it. As explained above, the resistive element may be a coiled metal wire or may be in the form of a thin film, thick film or foil resistor associated with a chip resistor.

In accordance with the present invention, the first and second electrically conductive elements serve two functions. A first function is to deliver electrical power to the heater device. A second function is to form a part of a divided junction thermocouple that monitors a temperature of the heater. A temperature of the heater is controlled by alternately coupling the first and second electrically conductive elements to a power source and to a temperature sensing electronic circuit. When the electrically conductive elements are coupled to the temperature sensing circuit, a controller associated with the temperature sensing circuit uses the sensed temperature to determine when the first and second electrically conductive elements are to be coupled to the power source. According to some implementations, the temperature sensing circuit comprises a voltage detector to which ends of the first and second electrically conductive elements are electrically couplable. Correlation methods well known in the art may be implemented by the controller to determine the temperature of the heater based on a voltage difference detected by the voltage detector and the temperature of the electronic circuit.

According to some implementations the systems and methods disclosed herein are for the purpose of delivering a therapeutic releasable device (e.g. an embolic coil) to a treatment site inside a patient (e.g. the site of an aneurysm). A pushwire and delivery catheter, in conjunction with other tools, are typically used in the delivery process with the pushwire being configured to carry the releasable device to the treatment site through a lumen of the delivery catheter.

One design includes first and second electrical conductors extending from the proximal end of the pushwire (the power supply end) to the distal end where the releasable implant and heater are located. As noted above, according to some implementations the heater includes a resistive element disposed between first and second electrical terminals to which the first and second electrical conductors are respectively electrically coupled. As also noted above, these electrical conductors are made of dissimilar metals and may comprise, for example, a nickel conductor and a stainless steel conductor. According to some implementations, a portion of the pushwire itself, which may be made of stainless steel, is used for at least a portion of one of the first and second electrical conductors. This advantageously makes it possible to use only one additional electrically conductive wire for connecting the heater to the power source and the thermocouple to the voltage detector that forms a part of the temperature sensing circuit. According to this example configuration, there will be a dissimilar junction at one side of the heater to the stainless steel conductive element and another dissimilar junction at the other side of the heater to the nickel conductive element. At the proximal end of the pushwire assembly this combination, which acts like a thermocouple, can be measured by well-known circuitry that includes a voltage detector as discussed above. The temperature reported will be in the middle of the temperatures at the electrical terminals of the heater where the dissimilar junctions occur. It does not matter what the material of the heater is as long as it is electrically conductive. For example, it can be platinum or another metal or a thick film resistor material.

In instances when the elongated shaft comprises a pushwire, the pushwire may be configured in a variety of ways. According to one implementation, the pushwire comprises a hypotube along substantially its entire length with a distal end electrically coupled to one of the electrical terminals of the heater. According to such an implementation, a length of the one additional electrically conductive wire may pass through an inner lumen of the hypotube or may alternatively run entirely external to the hypotube. According to another implementation, the pushwire comprises a solid core wire having a distal end electrically coupled to one of the electrical terminals of the heater, and the one additional electrically conductive wire runs external to the core wire. According to yet another implementation, the pushwire comprises a hypotube having a distal end to which a solid core wire is mechanically and electrically coupled. According to one such implementation, a distal end portion of the core wire is tapered with a distal end of the core wire being electrically coupled to one of the electrical terminals of the heater. It should be pointed out that pushwires used to delivery an embolic coil to the site of an aneurysm are relatively long (180 cm in some instances) and have a very small diametric profiles (e.g. 0.25 millimeters).

It is noted that adding multiple additional wires beyond what is needed to supply heater power complicates the design and adds cost to the delivery system and may not even be possible as they must fit in the very small diameter lumens involved.

An advantage in applications involving the implantation of therapeutic devices inside the human body is that the starting temperature of the resistor device/heater and electrically conductive elements prior to energizing the heater is always at body temperature at about 37 degrees C. Many errors can be eliminated in determining a temperature of the resistor device/heater because the starting temperature can be assumed to be within three degrees of 37 degrees C. For example, the output of the temperature sensing circuit can be measured prior to application of power to the heater, and this represents an initial value at approximately 37 degrees C. Then a setpoint can be determined as a delta from this initial value. This delta represents a specific temperature difference of the heater from the approximately 37 degrees. When the heater is subsequently powered to reach and be maintained at the setpoint it will thus be maintained at a specific absolute temperature which is a delta from the approximately 37 degrees.

According to one implementation a system is provided that includes an elongated shaft having a distal end portion to which a releasable device is coupled by a thermally severable connector. The system further includes a resistor device/heater that includes a first terminal, a second terminal and a resistive element disposed between and electrically coupled to the first and second terminals. The thermally severable connector is located adjacent to or in contact with at least a portion of the resistive element. First and second electrically conductive elements, that each have a first end and a second end, are used in connecting the resistor device/heater to a power source and alternatively to a voltage detector that forms a part of a temperature sensing circuit. The first ends of the first and second electrically conductive elements are electrically couplable to the power source, and the second ends of the first and second electrically conductive elements are respectively coupled to the first and second terminals of the resistor device/heater. The first electrically conductive element is made of a first metal and the second electrically conductive element is made of a second metal that is different than the first metal. The first and second electrically conductive elements are configured such that when there is a temperature difference between their respective first and second ends, a voltage is induced between the first and second electrically conductive elements that is proportional to the temperature difference.

The system may further comprise a control circuit that is configured to control a temperature of the resistor device/heater to a target temperature by alternately electrically coupling the first ends of the first and second electrically conductive elements to the power source and to a voltage detector with the first ends being repeatedly electrically coupled to the voltage detector. The control circuit is configured such that when the voltage detected by the voltage detector is above or at a target voltage that corresponds to the target temperature of the resistor device/heater, the first ends of the first and second electrically conductive elements are maintained electrically coupled to the voltage detector until the voltage detected by the voltage detector is below the target voltage, at which time the first ends are electrically decoupled from the voltage detector and electrically coupled to the power source. The target temperature of the resistor device/heater is selected to be sufficient to cause the thermally severable connector to sever.

When the first and second electrically conductive elements have been electrically coupled to the power source for the purpose of increasing the temperature of the heater, thereafter the control circuit momentarily and repeatedly couples the first ends of the first and second electrically conductive elements to the voltage detector to measure the heater temperature. The repeated and momentary connection of the first ends to the voltage detector continues until the target temperature of the heater is achieved, wherein thereafter, the first ends remain connected to the voltage detector until the detected temperature of the heater again falls below the target temperature.

Some metal conductors that are used in traditional thermocouple applications are not suitable for use in some of the devices and systems disclosed herein. In applications of the current invention, the first and second electrically conductive elements that form a part of the divided junction thermocouple are not only used to carry currents on the order of microamperes when measuring temperature, but must also carry much higher currents (e.g. 10-200 milliamperes) for the purpose of powering the heater. Moreover, when an electrically conductive element is in the form of a wire, the gauge of the wire used is very small (typically 43 AWG or smaller). Metal conductors that have high electrical resistivity (e.g. 0. 5 micro-ohm-meter), such as constantan, are not suitable for conducting the higher heater currents found in thermal embolic coil delivery systems where the length of the wires are long and their diameters are very small. One concern with using, for example, one copper wire and one constantan wire is that the electrical resistivity of constantan is approximately thirty times that of copper. This means that for the constantan wire to have the same electrical resistance of its copper neighbor, to carry the current needed for the heater, it needs to be about five times the diameter of the copper wire. Very fine, high gauge number copper wires (e.g. 43 ga.) are already being used to power such heaters in order to fit into the small catheter lumen, and the resistance of the existing art copper wire, one half of the two wire circuit, is typically 12 ohms. So having a much larger diameter constantan wire would be a problem getting it to fit. For this reason, according to some implementations, the electrically conductive elements that provide power to the heater are made of other lower resistivity metals, such as nickel which has a resistivity that is around four times that of copper.

Although wound-wire heaters and chip resistors are suitable for applying thermal energy to a thread to cause it to sever, chip resistors have a number of advantages over wound-wire heaters. First, because the coil of a coiled wire heater is typically made of a platinum alloy, the material cost is significant and the fabrication cost in winding it is also significant. Chip resistors are much less expensive and typically cost pennies. Another advantage is that chip resistors are available in a huge number of resistance values that facilitate optimization when designing systems that incorporate such devices. Chip resistors can also be significantly shorter than wound platinum alloy heaters. Wound-wire heaters on the market today have lengths between 0.6 to 1. I millimeters. Chip resistors, on the other hand, can have lengths of 0.3 to 0.4 millimeters (including its terminals). Moreover, larger resistance is possible with chip resistors which means less power is wasted in the resistance of the connected wires and more power is delivered to the heater itself This is highly beneficial when the power source is a battery.

These and other advantages and features will become apparent in view of the figures and of the detailed description.

### BRIEF DECSRIPTION OF THE DRAWINGS

FIG. I illustrates a prior art heater configuration.
FIG. 2 shows a heater device according to one implementation.
FIG. 3A illustrates a side view of a surface-mount device resistor according to one implementation.
FIG. 3B is a top view of the surface-mount device resistor of FIG. 3A
FIG. 4 illustrates a system for severing a connector that couples a releasable device to an end portion of an elongated shaft according to a first implementation.
FIG. 5 illustrates a system for severing a connector that couples a releasable device to an end portion of an elongated shaft according to a second implementation.
FIG. 6 illustrates a system for severing a connector that couples a releasable device to an end portion of an elongated shaft according to a third implementation.
FIG. 7 illustrates a system for severing a connector that couples a releasable device to an end portion of an elongated shaft according to a fourth implementation.
FIG. 8 illustrates a system for severing a connector that couples a releasable device to an end portion of an elongated shaft according to a fifth implementation.
FIG. 9A and 9B illustrate a control circuit that is configured to alternately couple the first ends of first and second electrically conductive elements made of dissimilar metals to a power source and a voltage detector.

### DETAILED DESCRIPTION

The description that follows is directed primarily to thermally severing a connector that couples an embolic coil to an end portion of a pushwire with the use of a resistor device that is adj acent to or in thermal contact with the connector. It is appreciated that the scope of the invention is not limited to embolic coil delivery systems, but is applicable to any system in which it is desirable to release a device from an end of an elongated shaft.

It is important to note that the drawings are not intended to depict the system components in precise detail, but are instead intended to show a general arrangement of the components for the purpose conveying how the components collectively function in the quest of thermally severing a connector that holds a releasable device to an end of an elongated shaft. It is also to be noted that the components depicted in the drawings are not to scale.

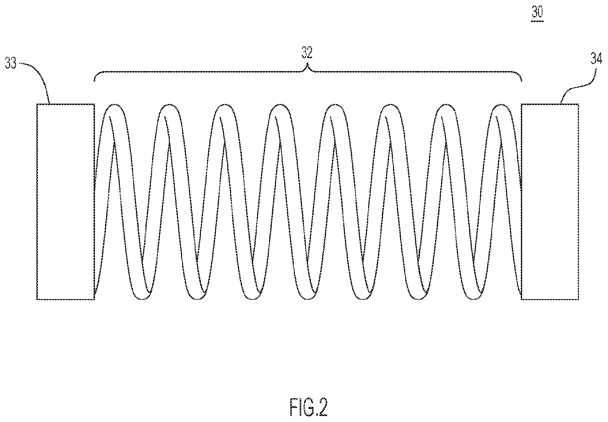

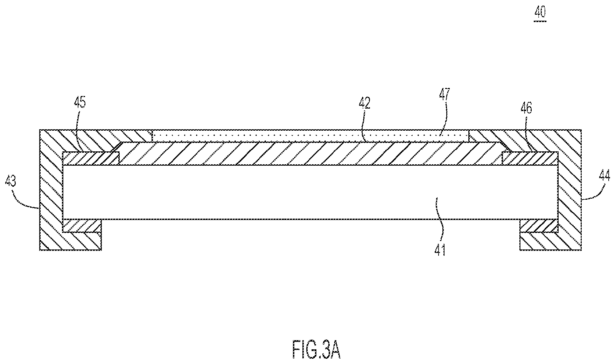

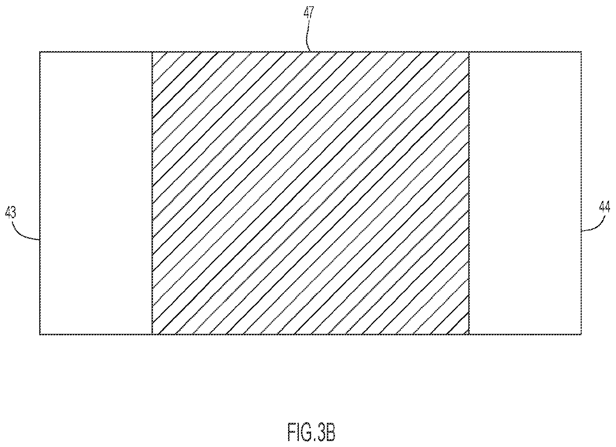

In the description that follows several types of heaters are disclosed for use in thermally severing a connector (e.g. a polymeric thread/wire) that tethers a releasable device (e.g. embolic coil) to an end portion of an elongated shaft (e.g. a pushwire). According to some implementations, the heater is a wound-wire heater 30, as shown in FIG. 2, which comprises a coiled resistive element 32 disposed between and electrically coupled to first and second electrically conductive terminals 33 and 34. According to some implementations, the resistive element comprises a platinum alloy wire. According to other implementations, the heater is a surface-mount device resistor (referred to herein as a "chip resistor). FIGS. 3A and 3B illustrate an example chip resistor 40 that includes a ceramic substrate 41 having a top surface on which is supported a resistive layer 42. As explained above, the resistive layer may comprise any of a number of configurations. Disposed at opposite ends of the resistive layer 42 are first and second electrically conductive terminals 43 and 44 that are respectively electrically coupled to the resistive layer by electrodes 45 and 46. The chip heater also typically includes a protective electrically insulated overcoat 47 atop the resistive layer 42. An example chip resistor suitable for the applications disclosed herein is one manufactured by Yageo under part number RC0075FS-7N200RP. As will be discussed in more detail below, in use the first and second electrically conductive terminals of each of heaters 30 and 40 are couplable to a power source 120 that induces current flow through their resistive elements 32 and 42 when their electrically conductive terminals are coupled to the power source.

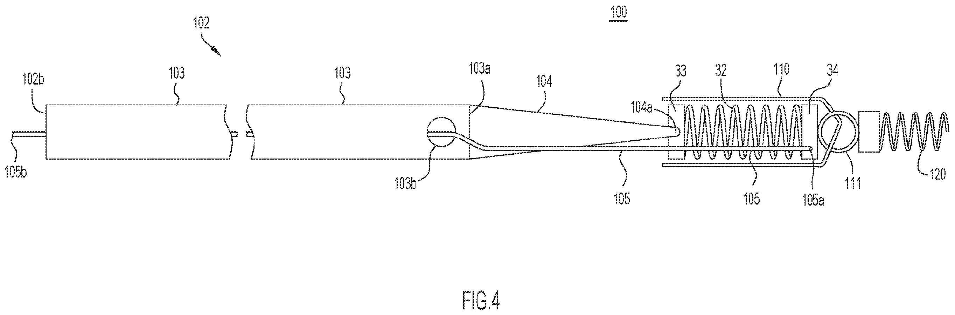

FIG. 4 illustrates a portion of an embolic coil delivery system 100 that utilizes a wound-wire heater 30 like that shown in FIG. 2. The system includes an electrically conductive pushwire 102 that comprises a hypotube 103 having a distal end 103a to which a solid core wire 104 is mechanically and electrically coupled. The distal end portion of the core wire 104 may or may not be tapered, but nonetheless includes a distal end 104a that is electrically coupled to the first electrically conductive terminal 33 of heater 30. An additional electrically conductive element 105 in the form of a wire has a distal end 105a that is electrically coupled to the second electrically conductive terminal 34 of heater 30. Pushwire 102 and wire 105 are electrically insulated from each other, with each including a proximal end that is connected to a control circuit 700 that is configured to control a temperature of the heater 30. Further, according to some implementations at least a portion of the length of the wire 105 passes through an internal lumen of the hypotube 103 and enters the internal lumen through an opening 103b preferably located along a distal end portion of the hypotube. According to other implementations the wire 105 may entirely reside outside the hypotube 103.

According to one implementation, each of the hypotube 103 and core wire 104 of the pushwire 102 comprises stainless steel and the additional electrically conductive element 105 comprises nickel. The embolic coil 120 is mechanically coupled to a distal end portion of the core wire 104 by a thermally severable connector 110 (e.g. a polymeric thread) that extends through a retention ring 111 associated with the embolic coil. In FIG. 4 the severable connector 110 is located adjacent the resistive element 32 of heater 30. According to other implementations the severable connector 110 is in physical contact with the resistive element 32. The goal is to control the temperature of the heater 30 to a target temperature (or a range of temperatures) that is sufficient to cause the thermally severable connector 110 to sever as the embolic coil 120 is introduced into an aneurysm.

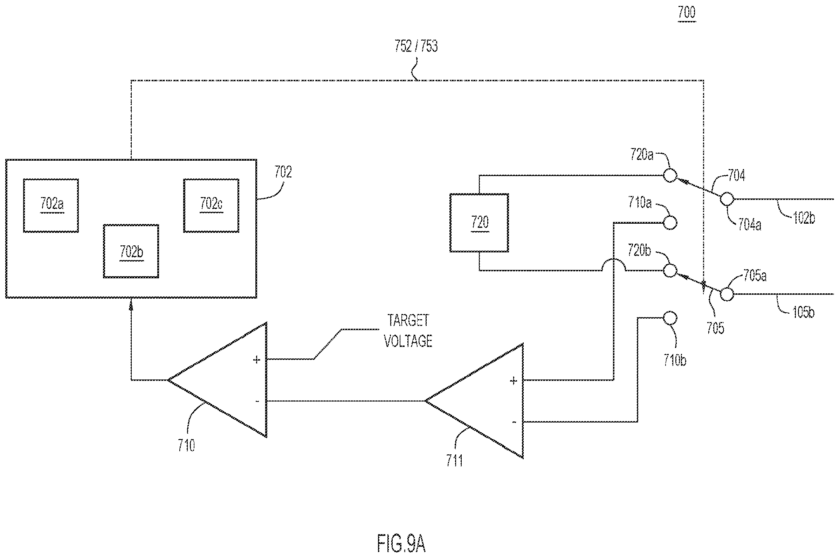

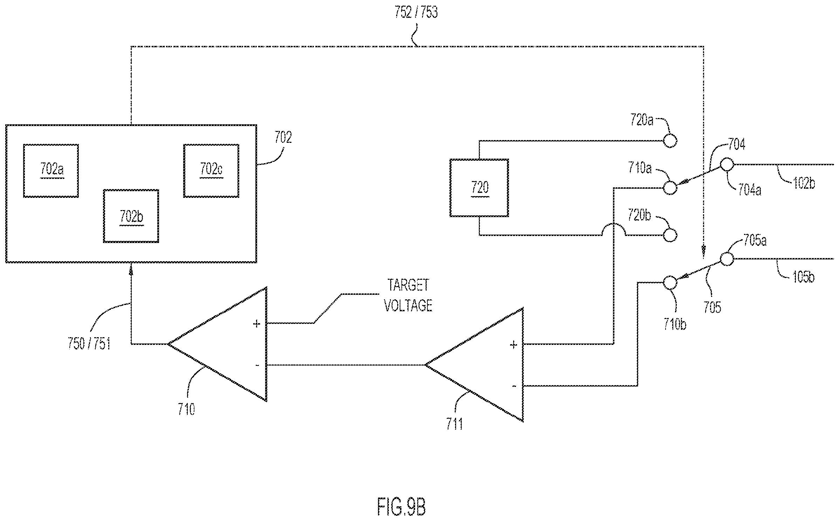

A salient feature of the systems and methods disclosed herein is that the electrically conductive elements that provide power to the heater are made of dissimilar metals. This allows the electrical conductors to serve two functions. A first function is to deliver electrical power to the heater terminals. A second function is to form a part of a divided junction thermocouple that is used to monitor a temperature of the heater. This allows the temperature of the heater to be controlled by a control circuit 700 like that depicted in FIGS. 9A and 9B. The control circuit 700 is configured alternately couple the proximal ends of the electrically conductive elements 102 and 105 to a power source 720 and to a voltage detector 710.

With particular reference to FIGS. 4 and 9A-B, the proximal ends (or proximal end portions) 102b and 105b of the pushwire 102 and wire 105 are respectively coupled to inlet terminals 704a and 705a of switches 704 and 705 in the control circuit 700. Switches 704 and 705 are controlled by a controller 702 t0 transition between first and second states. The first state being depicted in FIG. 9A in which switches 704 and 705 respectively couple the proximal ends 102b and 105b of the pushwire 102 and wire 105 to terminals 720a and 720b of the power source 720. The second state being depicted in FIG. 9B in which switches 704 and 705 respectively couple the proximal ends 102b and 105b of the pushwire 102 and wire 105 to terminals 71 Oa and 71 Ob of the voltage detector 710.

The control circuit 700 is configured to repeatedly electrically couple the pushwire 102 and wire 105 to the voltage detector 710 in order that the temperature of the heater 30 may be monitored to determine if it is operating above or below the target temperature. The control circuit 700 is configured such that when the voltage detected by the voltage detector 710 is above or at a target voltage that corresponds to the target temperature, switches 704 and 705 assume their second state to connect the pushwire 102 and wire 105 to the voltage detector 710. The switches 704 and 705 remain in their second state until the voltage detected by the voltage detector is below the target voltage at which time the switches transition to their first state to electrically couple the pushwire 102 and wire 105 to the power source 720.

When the switches 704 and 705 have assumed their first state, the control circuit 700 is configured to momentarily and repeatedly transition the switches to their second state for the purpose of monitoring the heater 30 temperature as the temperature is ramped up. The repeated and momentary transitioning of the switches 704 and 705 to their second state continues until the target temperature of the heater is achieved, wherein thereafter, the switches 704 and 705 remain in their second state until the detected temperature of the heater falls below the target temperature.

In the implementation of FIGS. 9A and 9B, when the switches 704 and 705 are in their second state, the electrically conductive elements 102 and 105 are coupled to a voltage amplifier 711 that amplifies the voltage induced between the proximal ends of the pushwire 102 and wire 105. The output of the voltage amplifier 711 is inputted to the voltage detector 710 where the amplified volage is compared to, for example, a target voltage corresponding to the target temperature of the heater 30. According to one implementation, upon the input voltage to the voltage detector 710 being less than the target voltage (which indicates the temperature of the heater 30 is below the target temperature), an output signal 750 is generated and delivered to the controller 702 where it is processed in a way that results in the controller outputting a control signal 752 that causes the switches 704 and 705 to assume their first state to enable the power source to activate the heater 30. If on the other hand, upon the input voltage to the voltage detector 710 being equal to or greater than the target voltage (which indicates the temperature of the heater 30 is at or above the target temperature), another output signal 751 is generated and delivered to the controller 702 where it is processed in a way that results in the controller outputting another control signal 753 that causes the switches 704 and 705 to assume or remain in their second state.

According to some implementations the controller 702 includes a processor 702a and a memory 702b that stores instructions to be executed by the processor. The processor 702a, through the use of data and/or instructions stored in memory 702b, processes signals 750/751 to determine the type of control signal 752/753 to be outputted by the controller. According to some implementations, the controller 702 also includes a clock 702c that is useable by the controller to switch the switches 704 and 705 between their first and second states at a fixed rate (e.g. 200 Hz rate) with a duty cycle such that the power source is coupled to the pushwire 102 and wire 105 for 4. 5 milliseconds of every duty cycle and such that the thermocouple (formed by pushwire 102 and wire 105) is coupled to the voltage detector for 0. 5 millisecond of every duty cycle. Other duty cycles and intervals are also contemplated.

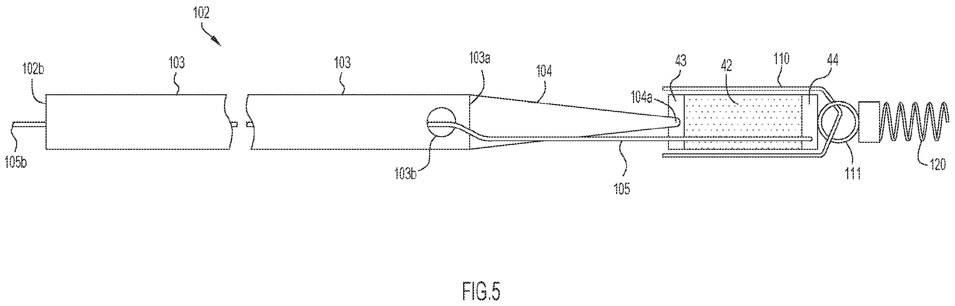

The system of FIG. 5 is similar to the system of FIG. 4 with the exception that the heater is a chip resistor 40 like that depicted in FIGS. 3A and 3B, and the distal end 104a of the core wire is coupled to electrically conductive terminal 43 and the distal end 105a of wire 105 is coupled to electrically conductive terminal 44.

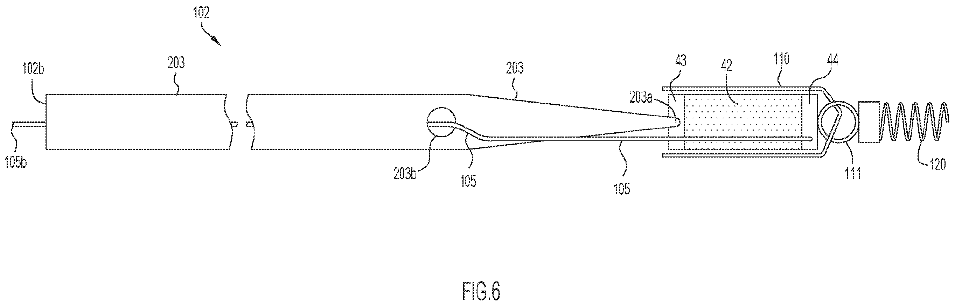

The system of FIG. 6 is a variant of the system of FIG. 5, wherein the pushwire 102 comprises a hypotube 203 along substantially its entire length with a distal end 203a being coupled to the electrically conductive terminal 43 of heater 40. Additionally, the distal end 105a of wire 105 is coupled to electrically conductive terminal 44 of heater 40. According to such an implementation, a length of the wire 105 may pass through an inner lumen of the hypotube 203 or may alternatively run entirely external to the hypotube. In instances where at least a portion of the length of the wire 105 passes through the inner lumen of the hypotube 203, the wire may enter the inner lumen through a side opening 203b or other opening of the hypotube.

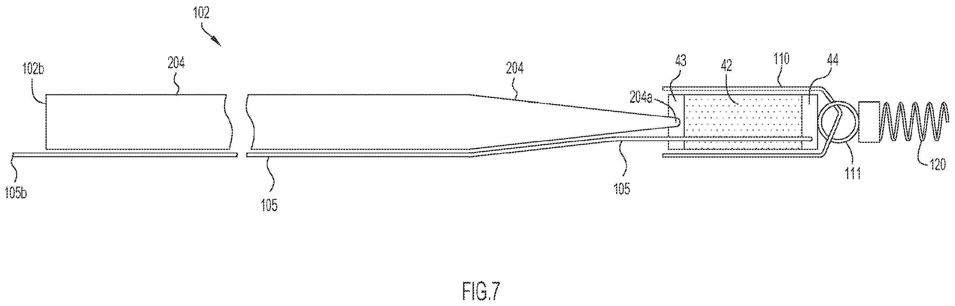

According to another implementation, as shown in FIG. 7, the entirety of the pushwire 102 comprises a solid core wire 204 having a distal end 204a coupled to the electrically conductive terminal 43 of heater 40. Additionally, the distal end 105a of wire 105 is coupled to the electrically conductive terminal 44 of heater 40.

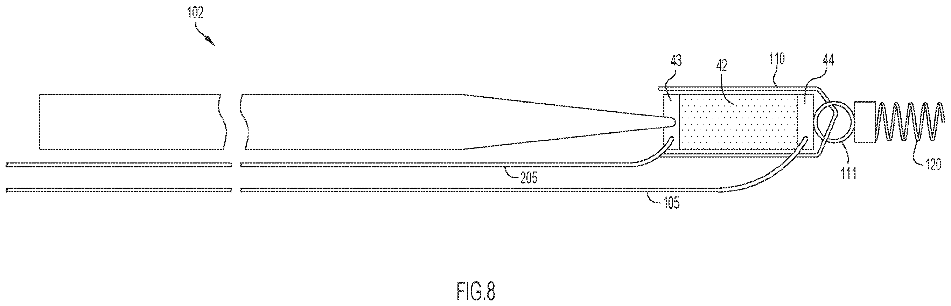

In the forgoing examples, the pushwire 102 is electrically conductive with a distal end being coupled to one of the electrically conductive terminals of heater 30 (FIG. 4) or heater 40 (FIGS. 5- 7). However, according to other implementations, like the system illustrated in FIG. 8, a pair of electrically conductive wires 205 and 105 made of dissimilar metals are respectively coupled to heater terminals 43 and 44 to the inlet terminals 704a and 705a of switches 704 and 705 located in the control circuit 700. In the implementation of FIG. 8, wire 205 is used in lieu of the pushwire 102 to deliver power to the heater 40 when switches 704 and 705 are in their first state. Wire 205 is also used in lieu of the pushwire 102 to form with wire 105 a thermocouple when switches 704 and 705 are in their second state. In all other respects the temperature of the heater 40 is regulated in accordance with one or more of the control schemes disclosed above.

It should be recognized that the scope of the invention is not limited to the particular implementations disclosed herein, and that various modifications can be made without departing from the scope of the invention as defined by the claims. For example, the invention is not limited to the electrically conductive materials disclosed herein, nor is it limited to the arrangements depicted in the drawings. Additionally, the invention encompasses heater types other than wound-wire types and chip resistor types. Moreover, it is appreciated that controllers other than the type disclosed herein may be used to implement the control schemes disclosed herein.

## Claims

1. A system (100) comprising:
an elongated shaft having a distal end portion;
a releasable device (120) coupled to the distal end portion of the elongated shaft by a thermally severable connector (110);
**characterized by**:
a surface-mount device resistor (40) including a ceramic substrate (41) having a surface on which is supported a resistive layer (42), disposed at opposite ends of which layer are first (43) and second (44) electrically conductive terminals respectively electrically coupled to the resistive layer (42), the thermally severable connector (110) being located adjacent to or in thermal contact with at least a portion of the resistive layer (42) such that when the resistive layer (42) is at a target temperature the thermally severable connector (110) is caused to sever; and
a first electrically conductive element (102) being made of a first metal having a first Seebeck coefficient and second electrically conductive element (105) that is made of a second metal having a second Seebeck coefficient different to the first Seebeck coefficient, each of the first (102) and second (105) conductive elements having a first end and a second end, the first ends of the first (102) and second (105) electrically conductive elements being alternately electrically couplable to a power source (720) and a voltage detector (710), the second ends of the first (102) and second (105) electrically conductive elements being respectively coupled to the first and second electrically conductive terminals of the surface-mount device resistor (40) to form a divided junction thermocouple configured to produce an output voltage at the first ends of the first (102) and second (105) electrically conductive elements that is proportional to the temperature difference between their respective first and second ends.

2. The system (100) according to claim 1, further comprising a control circuit (700) that is configured to control a temperature of the surface-mount device resistor (40) to a target temperature by alternately electrically coupling the first ends of the first (102) and second (105) electrically conductive elements to the power source (720) and to the voltage detector (710), the first ends being repeatedly electrically coupled to the voltage detector (710), the control circuit (700) being configured such that when the output voltage detected by the voltage detector (710) is above or at a target voltage that corresponds to the target temperature, the first ends of the first (102) and second (105) electrically conductive elements are maintained electrically coupled to the voltage detector (710) until the output voltage detected by the voltage detector (710) is below the target voltage at which time the first ends are electrically decoupled from the voltage detector (710) and electrically coupled to the power source (720), the target temperature of the resistor device being sufficient to cause the thermally severable connector (110) to sever.

3. The system (100) according to claim 2, wherein upon the first (102) and second (105) electrically conductive elements being electrically coupled to the power source (720) for the purpose of increasing the temperature of the resistive layer (42), the control circuit (700) is configured to momentarily and repeatedly couple the first ends of the first (102) and second (105) electrically conductive elements to the voltage detector (710) to measure the surface-mount device resistor temperature, the repeated and momentary connection of the first ends of the first (102) and second (105) electrically conductive elements to the voltage detector (710) continues until the target temperature is achieved, wherein thereafter, the first ends of the first ends of the first (102) and second (105) electrically conductive elements remain connected to the voltage detector (710) until the measured temperature of the surface-mount device resistor (40) falls below the target temperature.

4. The system (100) according to claim 1, wherein the surface-mount device resistor (40) includes a protective electrically insulated overcoat (47) atop the resistive layer (42).

5. The system (100) according to claim 1, wherein the first electrically conductive element (102) comprises stainless steel and the second electrically conductive element comprises nickel.

6. The system (100) according to claim 2, wherein the control circuit (700) includes a controller (702) that is configured to control a state of the first and second electrically actuated switches, each of the first and second electrically actuated switches being transitional between a first state and a second state, when in their first state the first and second electrically actuated switches electrically couple the first ends of the first (102) and second (105) electrically conductive elements to the power source (720), when in their second state the first and second electrically actuated switches electrically couple the first ends of the first (102) and second (105) electrically conductive elements to the voltage detector (710).

7. The system (100) according to claim 6, wherein the controller (702) is configured to cause:
the first and second electrically actuated switches to assume their first state at times when the output voltage detected by the voltage detector (710) is below the target voltage, and
the first and second electrically actuated switches to maintain their second state when the output voltage detected by the voltage detector (710) is at or above the target voltage.

8. The system (100) according to claim 7, wherein the controller (702) is configured to cause the first and second electrically actuated switches to maintain their second state when the voltage detected by the voltage detector (710) is at or above the target voltage.

9. The system (100) according to claim 1, wherein the elongated shaft is at least a part of one of the first (102) and second (105) electrically conductive elements.

10. The system (100) according to claim 1, wherein the elongated shaft is a push wire of an embolic coil delivery device, and the releasable device is an embolic coil.

11. The system (100) according to claim 1, wherein the thermally severable connector (110) is located adjacent to or in thermal contact with at least a portion of the resistive layer (42) such that when power is delivered to the surface-mount device resistor (40) the resistive layer (42) produces heat sufficient to thermally sever the thermally severable connector (110).

## Patentansprüche

1. Ein System (100), umfassend:
einen länglichen Schaft mit einem distalen Endabschnitt;
eine lösbare Vorrichtung (120), die durch einen thermisch abtrennbaren Verbinder (110) an den distalen Endabschnitt des länglichen Schafts gekoppelt ist;
**gekennzeichnet durch**:
einen Oberflächenmontagevorrichtungswiderstand (40), der ein Keramiksubstrat (41) mit einer Oberfläche aufweist, auf der eine Widerstandsschicht (42) getragen wird, wobei an gegenüberliegenden Enden der Schicht erste (43) und zweite (44) elektrisch leitende Anschlüsse angeordnet sind, die jeweils elektrisch mit der Widerstandsschicht (42) gekoppelt sind, wobei der thermisch abtrennbare Verbinder (110) benachbart zu oder in thermischem Kontakt mit mindestens einem Abschnitt der Widerstandsschicht (42) angeordnet ist, so dass, wenn die Widerstandsschicht (42) eine Zieltemperatur aufweist, der thermisch abtrennbare Verbinder (110) zum Abtrennen veranlasst wird; und
ein erstes elektrisch leitfähiges Element (102) aus einem ersten Metall mit einem ersten Seebeck-Koeffizienten und ein zweites elektrisch leitfähiges Element (105), das aus einem zweiten Metall mit einem zweiten Seebeck-Koeffizienten, der sich von dem ersten Seebeck-Koeffizienten unterscheidet, hergestellt ist, wobei jedes der ersten (102) und zweiten (105) leitfähigen Elemente ein erstes Ende und ein zweites Ende aufweist, wobei die ersten Enden der ersten (102) und zweiten (105) elektrisch leitfähigen Elemente abwechselnd elektrisch an eine Stromquelle (720) und einen Spannungsdetektor (710) koppelbar sind, wobei die zweiten Enden der ersten (102) und zweiten (105) elektrisch leitfähigen Elemente jeweils an den ersten und zweiten elektrisch leitfähigen Anschluss des Oberflächenmontagevorrichtungswiderstands (40) gekoppelt sind, um ein geteiltes Übergangsthermoelement zu bilden, das dazu konfiguriert ist, eine Ausgangsspannung an den ersten Enden der ersten (102) und zweiten (105) elektrisch leitfähigen Elemente zu erzeugen, die proportional zu der Temperaturdifferenz zwischen ihren jeweiligen ersten und zweiten Enden ist.

2. Das System (100) nach Anspruch 1, ferner umfassend eine Steuerschaltung (700), die konfiguriert ist, um eine Temperatur des Oberflächenmontagevorrichtungswiderstands (40) auf eine Zieltemperatur zu steuern, indem die ersten Enden des ersten (102) und des zweiten (105) elektrisch leitfähigen Elements abwechselnd mit der Stromquelle (720) und mit dem Spannungsdetektor (710) elektrisch gekoppelt werden, wobei die ersten Enden wiederholt elektrisch mit dem Spannungsdetektor (710) gekoppelt werden, wobei die Steuerschaltung (700) so konfiguriert ist, dass, wenn die vom Spannungsdetektor (710) erfasste Ausgangsspannung über oder bei einer Zielspannung liegt, die der Zieltemperatur entspricht, das erste Ende des ersten (102) und des zweiten (105) elektrisch leitfähigen Elemente mit dem Spannungsdetektor (710) elektrisch gekoppelt gehalten werden, bis die vom Spannungsdetektor (710) erfasste Ausgangsspannung unter der Zielspannung liegt, und zu diesem Zeitpunkt die ersten Enden vom Spannungsdetektor (710) elektrisch entkoppelt und mit der Stromquelle (720) elektrisch gekoppelt werden, wobei die Zieltemperatur der Widerstandsvorrichtung ausreicht, um eine Abtrennung des thermisch abtrennbaren Verbinders (110) zu veranlassen.

3. Das System (100) nach Anspruch 2, wobei, wenn das erste (102) und das zweite (105) elektrisch leitende Elemente elektrisch an die Stromquelle (720) gekoppelt sind, um die Temperatur der Widerstandsschicht (42) zu erhöhen, die Steuerschaltung (700) dazu konfiguriert ist, die ersten Enden des ersten (102) und des zweiten (105) elektrisch leitenden Elements vorübergehend und wiederholt an den Spannungsdetektor (710) zu koppeln, um die Oberflächenmontagevorrichtungswiderstandstemperatur zu messen, die wiederholte und vorübergehende Verbindung der ersten Enden des ersten (102) und des zweiten (105) elektrisch leitenden Elements mit dem Spannungsdetektor (710) fortgesetzt wird, bis die Zieltemperatur erreicht ist, wobei danach die ersten Enden der ersten Enden des ersten (102) und des zweiten (105) elektrisch leitenden Elements mit dem Spannungsdetektor (710) verbunden bleiben, bis die gemessene Temperatur des Oberflächenmontagevorrichtungswiderstands (40) unter die Zieltemperatur fällt.

4. Das System (100) nach Anspruch 1, wobei der Oberflächenmontagevorrichtungswiderstand (40) einen elektrisch isolierten Schutzüberzug (47) auf der Widerstandsschicht (42) beinhaltet.

5. Das System (100) nach Anspruch 1, wobei das erste elektrisch leitende Element (102) rostfreien Stahl umfasst und das zweite elektrisch leitende Element Nickel umfasst.

6. Das System (100) nach Anspruch 2, wobei die Steuerschaltung (700) eine Steuerung (702) beinhaltet, die dazu konfiguriert ist, einen Zustand des ersten und des zweiten elektrisch betätigten Schalters zu steuern, wobei der erste und der zweite elektrisch betätigte Schalter jeweils zwischen einem ersten Zustand und einem zweiten Zustand umschaltbar sind, wobei der erste und der zweite elektrisch betätigte Schalter in ihrem ersten Zustand die ersten Enden des ersten (102) und zweiten (105) elektrisch leitenden Elemente mit der Stromquelle (720) elektrisch verbinden, während der erste und der zweite elektrisch betätigte Schalter in ihrem zweiten Zustand die ersten Enden des ersten (102) und zweiten (105) elektrisch leitenden Elements mit dem Spannungsdetektor (710) elektrisch verbinden.

7. Das System (100) nach Anspruch 6, wobei die Steuerung (702) so konfiguriert ist, dass sie bewirkt, dass:
der erste und der zweite elektrisch betätigte Schalter ihren ersten Zustand zu Zeiten einnehmen, zu denen von dem Spannungsdetektor (710) erfasste Ausgangsspannung unter der Zielspannung liegt, und
der erste und der zweite elektrisch betätigte Schalter ihren zweiten Zustand beibehalten, wenn die von dem Spannungsdetektor (710) erfasste Ausgangsspannung bei oder über der Zielspannung liegt.

8. Das System (100) nach Anspruch 7, wobei die Steuerung (702) dazu konfiguriert ist, den ersten und zweiten elektrisch betätigten Schalter zu veranlassen, ihren zweiten Zustand beizubehalten, wenn die durch den Spannungsdetektor (710) erfasste Spannung bei oder über der Zielspannung liegt.

9. Das System (100) nach Anspruch 1, wobei die längliche Welle mindestens ein Teil von einem von dem ersten (102) und dem zweiten (105) elektrisch leitfähigen Element ist.

10. Das System (100) nach Anspruch 1, wobei die längliche Welle ein Schubdraht einer Vorrichtung zum Einbringen einer Emboliespule ist und die lösbare Vorrichtung eine Emboliespule ist.

11. Das System (100) nach Anspruch 1, wobei der thermisch abtrennbare Verbinder (110) benachbart zu oder in thermischem Kontakt mit mindestens einem Abschnitt der Widerstandsschicht (42) angeordnet ist, so dass, wenn Strom an den Oberflächenmontagevorrichtungswiderstand (40) geliefert wird, die Widerstandsschicht (42) Wärme erzeugt, die ausreicht, um den thermisch abtrennbaren Verbinder (110) thermisch abzutrennen.

## Revendications

1. Un système (100) comprenant :
une tige allongée ayant une partie d'extrémité distale ;
un dispositif libérable (120) couplé à la partie d'extrémité distale de la tige allongée par un connecteur thermoséparable (110) ;
**caractérisé par**:
une résistance de dispositif à montage en surface (40) comprenant un substrat en céramique (41) ayant une surface sur laquelle est supportée une couche résistive (42), aux extrémités opposées de laquelle sont disposées des première (43) et seconde (44) bornes électriquement conductrices, couplées électriquement de manière respective à la couche résistive (42), le connecteur thermoséparable (110) étant situé adjacent à ou en contact thermique avec au moins une partie de la couche résistive (42) de telle sorte que lorsque la couche résistive (42) est à une température cible, le connecteur thermoséparable (110) est amené à se séparer ; et
un premier élément électriquement conducteur (102) étant fait d'un premier métal ayant un premier coefficient de Seebeck et un second élément électriquement conducteur (105) qui est fait d'un second métal ayant un second coefficient de Seebeck différent du premier coefficient de Seebeck, chacun des premier (102) et second (105) éléments conducteurs ayant une première extrémité et une seconde extrémité, les premières extrémités des premier (102) et second (105) éléments électriquement conducteurs étant alternativement couplables électriquement à une source d'alimentation (720) et à un détecteur de tension (710), les secondes extrémités des premier (102) et second (105) éléments électriquement conducteurs étant respectivement couplées aux première et seconde bornes électriquement conductrices de la résistance de dispositif à montage en surface (40) pour former un thermocouple à jonction divisée configuré pour produire une tension de sortie aux premières extrémités des premier (102) et second (105) éléments électriquement conducteurs qui est proportionnelle à la différence de température entre leurs première et seconde extrémités respectives.

2. Le système (100) selon la revendication 1, comprenant en outre un circuit de commande (700) qui est configuré pour commander une température de la résistance de dispositif à montage en surface (40) à une température cible en couplant électriquement de manière alternée les premières extrémités des premier (102) et second (105) éléments électriquement conducteurs à la source d'alimentation (720) et au détecteur de tension (710), les premières extrémités étant couplées électriquement de manière répétée au détecteur de tension (710), le circuit de commande (700) étant configuré de telle sorte que lorsque la tension de sortie détectée par le détecteur de tension (710) est supérieure à ou égale à la tension cible qui correspond à la température cible, les premières extrémités des premier (102) et second (105) éléments électriquement conducteurs sont maintenues électriquement couplées au détecteur de tension (710) jusqu'à ce que la tension de sortie détectée par le détecteur de tension (710) soit inférieure à la tension cible, moment auquel les premières extrémités sont électriquement découplées du détecteur de tension (710) et électriquement couplées à la source d'alimentation (720), la température cible du dispositif à résistance étant suffisante pour provoquer la séparation du connecteur thermoséparable (110).

3. Le système (100) selon la revendication 2, dans lequel, lorsque les premier (102) et second (105) éléments électriquement conducteurs sont électriquement couplés à la source d'alimentation (720) dans le but d'augmenter la température de la couche résistive (42), le circuit de commande (700) est configuré pour coupler momentanément et de manière répétée les premières extrémités des premier (102) et second (105) éléments électriquement conducteurs au détecteur de tension (710) pour mesurer la température de la résistance de dispositif à montage en surface, la connexion répétée et momentanée des premières extrémités des premier (102) et second (105) éléments électriquement conducteurs au détecteur de tension (710) continue jusqu'à ce que la température cible soit atteinte, dans lequel, par la suite, les premières extrémités des premières extrémités des premier (102) et second (105) éléments électriquement conducteurs restent connectées au détecteur de tension (710) jusqu'à ce que la température mesurée de la résistance de dispositif à montage en surface (40) tombe en dessous de la température cible.

4. Le système (100) selon la revendication 1, dans lequel la résistance de dispositif à montage en surface (40) comprend une surcouche de protection électriquement isolée (47) au sommet de la couche résistive (42).

5. Le système (100) selon la revendication 1, dans lequel le premier élément électriquement conducteur (102) comprend de l'acier inoxydable et le second élément électriquement conducteur comprend du nickel.

6. Le système (100) selon la revendication 2, dans lequel le circuit de commande (700) comprend un dispositif de commande (702) qui est configuré pour commander un état des premier et second commutateurs à actionnement électrique, chacun des premier et second commutateurs à actionnement électrique pouvant alterner entre un premier état et un second état, lorsque dans leur premier état, les premier et second commutateurs à actionnement électrique couplent électriquement les premières extrémités des premier (102) et second (105) éléments électriquement conducteurs à la source d'alimentation (720), lorsque dans leur second état, les premier et second commutateurs à actionnement électrique couplent électriquement les premières extrémités des premier (102) et second (105) éléments électriquement conducteurs au détecteur de tension (710).

7. Le système (100) selon la revendication 6, dans lequel le dispositif de commande (702) est configuré pour faire en sorte que :
les premier et second commutateurs actionnés électriquement prennent leur premier état à des moments où la tension de sortie détectée par le détecteur de tension (710) est inférieure à la tension cible, et
les premier et second commutateurs actionnés électriquement maintiennent leur second état lorsque la tension de sortie détectée par le détecteur de tension (710) est égale ou supérieure à la tension cible.

8. Le système (100) selon la revendication 7, dans lequel le dispositif de commande (702) est configuré pour faire en sorte que les premier et second commutateurs actionnés électriquement maintiennent leur second état lorsque la tension détectée par le détecteur de tension (710) est égale ou supérieure à la tension cible.

9. Le système (100) selon la revendication 1, dans lequel la tige allongée fait au moins partie de l'un des premier (102) et second (105) éléments électriquement conducteurs.

10. Le système (100) selon la revendication 1, dans lequel la tige allongée est un fil de poussée d'un dispositif de distribution de bobine embolique, et le dispositif libérable est une bobine embolique.

11. Le système (100) selon la revendication 1, dans lequel le connecteur thermoséparable (110) est situé de façon adjacente à ou en contact thermique avec au moins une partie de la couche résistive (42) de sorte que lorsque la résistance de dispositif à montage en surface (40) est alimentée en courant, la couche résistive (42) génère suffisamment de chaleur pour provoquer la séparation thermique du connecteur thermoséparable (110).
